Europäisches Patentamt

European Patent Office

Office européen des brevets

(11) Veröffentlichungsnummer: **0 281 763 B1**

# (12) EUROPÄISCHE PATENTSCHRIFT

(45) Veröffentlichungstag der Patentschrift: **30.12.92**

(21) Anmeldenummer: **88101657.0**

(22) Anmeldetag: **05.02.88**

(51) Int. Cl.⁵: **A61B 17/56**

(54) **Hilfsinstrument zum Setzen von Löchern beim Implantieren von Verriegelungsnägeln.**

(30) Priorität: **07.03.87 DE 8703438 U**

(43) Veröffentlichungstag der Anmeldung:
**14.09.88 Patentblatt 88/37**

(45) Bekanntmachung des Hinweises auf die
Patenterteilung:
**30.12.92 Patentblatt 92/53**

(84) Benannte Vertragsstaaten:
**AT CH DE FR GB IT LI NL**

(56) Entgegenhaltungen:
**EP-A- 0 201 737**
**FR-A- 2 233 972**
**US-A- 4 364 381**

(73) Patentinhaber: **Howmedica GmbH**
**Professor-Küntscher-Str. 1-5**
**W-2314 Schönkirchen ü. Kiel(DE)**

(72) Erfinder: **Pennig, Dietmar, Dr. med.**
**Schmeddingstrasse 99**
**W-4400 Münster(DE)**
Erfinder: **Brug, Erwin, Prof. Dr.**
**Von-Esmarch-Strasse 133**
**W-4400 Münster(DE)**
Erfinder: **Harder, Hans Erich**
**Mecklenburger Strasse 35**
**W-2316 Probsteierhagen(DE)**

(74) Vertreter: **Dipl.-Ing. H. Hauck, Dipl.-Ing. E.**
**Graalfs, Dipl.-Ing. W. Wehnert, Dr.-Ing. W. Dö-**
**ring**
**Neuer Wall 41**
**W-2000 Hamburg 36(DE)**

Rank Xerox (UK) Business Services

## Beschreibung

Die Erfindung bezieht sich auf ein Hilfsinstrument zum Setzen von Löchern beim Implantieren von Verriegelungsnägeln gemäß dem Oberbegriff des Anspruchs 1.

Verriegelungsnägel sind bekanntlich in den Knochenkanal einführbare Knochennägel mit Querbohrungen. Die Verriegelungsnägel werden mit Hilfe von in die Querbohrungen eingeführten Knochenschrauben sowohl in Achs- als auch in Drehrichtung im Knochen fixiert. Diesem Vorgang geht das Setzen von Löchern im Knochen voraus. Hierzu wird der Knochen gleichachsig zu den Querbohrungen im Verriegelungsnagel aufgebohrt. Die Querbohrungen sind jedoch in ihrer Lage ohne besondere Hilfsmittel nicht exakt zu bestimmen. Hierfür werden Zielgeräte verwendet. Die meisten bekannten Zielgeräte benötigen hierzu eine energiereiche Strahlungsquelle, zum Beispiel eine Röntgenstrahlungsquelle sowie ein Empfangsgerät und einen Bildwandler.

Bei einer bekannten Kategorie von Zielgeräten werden diese an der Röntgenquelle angebracht. Die Erfindung betrifft demgegenüber ein freihändig vom Operateur bedienbares Zielgerät. Aus der EP-A-0 201 737 ist ein Hilfsinstrument zum Setzen von Löchern beim Implantieren von Verriegelungsnägeln (Zielgerät) bekanntgeworden, bei dem an einem länglichen Schaft an einem Ende ein Handgriff und am anderen Ende eine Zielvorrichtung angeordnet ist. Die Zielvorrichtung enthält ein Visier und eine von dem Visier seitlich versetzte Bohrhülse. In die Bohrhülse kann ein Zielstift eingeführt werden, der aus für Röntgenstrahlung durchlässigem Material besteht. An der Spitze des Stiftes kann röntgenstrahlungsundurchlässiges Material angebracht werden, um einen Markierungspunkt zu setzen. Mit Hilfe des Visiers und des Zielstiftes wird die Bohrhülse lagerichtig gegen den Knochen angelegt. Anschließend erfolgt nach erneuter Ausrichtung mit Hilfe des Visiers das Setzen des Loches, indem das Bohrwerkzeug über die Bohrhülse eingeführt wird. Das bekannte Instrument benötigt daher zwei Zielvorgänge nacheinander.

Der Erfindung liegt die Aufgabe zugrunde, ein Hilfsinstrument zum Setzen von Löchern beim Implantieren von Verriegelungsnägeln zu schaffen, das besonders leicht handhabbar ist und ein einfaches und zielgenaues Setzen der Löcher erlaubt.

Diese Aufgabe wird gelöst durch die Merkmale des Patentanspruchs 1.

Bei dem erfindungsgemäßen Hilfsinstrument sind zur Deckung bringbare Zielabschnitte und Führungsbohrung im für Röntgenstrahlung durchlässigen Führungsorgan koaxial. Die Führungsbohrung dient zur gleitenden Führung eines drahtähnlichen Stichwerkzeugs.

Die Erfindung geht von der Erkenntnis aus, daß es mit Hilfe eines geeigneten Stichwerkzeugs ohne weiteres möglich ist, die äußere Cortikalis durch eine sehr geringe Anzahl von Schlägen zu durchdringen. Das auf diese Weise gesetzte Loch ist exakt auf der Achse der Querbohrung. Die genaue Ausrichtung erfolgt mit Hilfe der Zielabschnitte, die auf dem Bildwandler anzeigen, wenn die Achse der Führungsbohrung mit der Achse der Querbohrung übereinstimmt. Anschließend, falls erwünscht, kann das mit Hilfe des Stichwerkzeugs erzeugte Loch auf die gewünschte Weise aufgebohrt werden. Gleichzeitig kann ein Loch in der gegenüberliegenden Cortikalis gebohrt werden.

Für das erfindungsgemäße Hilfsinstrument verwendbare Stichwerkzeuge sind bekannt, zum Beispiel als sogenannte Steinman-Pins. Derartige Stifte werden zum Beispiel dazu verwendet, um auf Knochensegmente eine Zugkraft aufzubringen oder um extern am Knochen eine Vorrichtung zu befestigen.

Über Bildwandlerbeobachtung kann die Übereinstimmung der Spitze des Stichwerkzeugs in der Führungsbohrung sehr leicht auf die Achse der Querbohrung im Verriegelungsnagel gebracht werden. Dies geschieht beispielsweise dadurch, daß zunächst nur die Spitze über dem Bildwandler an den richtigen Ort plaziert wird. Anschließend wird das Instrument verschwenkt, bis die Zielabschnitte als deckungsgleich auf dem Bildwandler abgebildet werden. Dadurch ist angezeigt, daß die Achse des Stichinstruments mit der Achse der Querbohrung im Verriegelungsnagel zusammenfällt. Durch einen einzigen oder durch mehrere rasche Schläge mit einem Hammer oder einem anderen Schlaginstrument auf das Stichinstrument wird ein zu den Querbohrungen ausgerichtetes Loch gesetzt.

Es sind verschiedene Möglichkeiten denkbar, die Zielabschnitte auszubilden. Eine besteht erfindungsgemäß darin, daß die Zielabschnitte von axial beabstandeten Ringen gebildet sind, die konzentrisch zur Führungsbohrung angeordnet sind.

Das Führungsorgan ist vorzugsweise aus Kunststoff geformt. Kunststoff ist bekanntlich strahlendurchlässig. Um den aus Kunststoff bestehenden Führungsabschnitt zu haltern, sieht eine Ausgestaltung der Erfindung vor, daß die Zielabschnitte als Fassung für das Führungsorgan dienen. Da die Führungsbohrung im Führungsorgan einer gewissen Belastung ausgesetzt ist, kann die Notwendigkeit bestehen, das Führungsorgan von Zeit zu Zeit auszuwechseln. Daher sieht eine weitere Ausgestaltung der Neuerung vor, daß mindestens ein Zielabschnitt lösbar am Schaft befestigt ist.

Eine alternative Ausführungsform sieht vor, daß Griff, Schaft und Führungsorgan einteilig aus Kunststoff geformt sind. Ein derartiges Teil läßt sich relativ unaufwendig mit geeigneten Formtechniken herstellen. Ist die Führungsbohrung durch häufigeren Gebrauch aufgeweitet, kann ein derartiges Instrument fortgeworfen und durch ein anderes ersetzt werden.

Eine andere Ausgestaltung der Erfindung sieht vor, daß die Zielvorrichtung im Abstand zur Führungsbohrung einen durchgehenden Schlitz oder dergleichen aufweist, der sich quer zur Führungsbohrung erstreckt. Ein derartiger Schlitz oder eine derartige Nut wird in ihrem Endbereich auf dem Bildwandler sichtbar, wenn das erfindungsgemäße Instrument nicht exakt ausgerichtet ist. Je nachdem, ob eine Abbildung an der oberen oder der unteren Seite auftaucht, erkennt der Operateur, nach welcher Seite er das Instrument verschwenken muß, um die gewünschte Ausrichtung zu erhalten.

Bei Verriegelungsnägeln sind üblicherweise die Querbohrungen paarweise angeordnet. Um ein erneutes Zielen und damit unter Umständen notwendiges Versetzen der Strahlungsanordnung zu vermeiden, sieht eine Ausgestaltung der Erfindung vor, daß im Abstand zur Führungsbohrung eine weitere Bohrung zur Aufnahme einer Bohrhülse vorgesehen ist, deren Achse parallel zur Achse der Führungsbohrung verläuft. Das Setzen des ersten Loches erfolgt in der oben beschriebenen Weise. Das Stichwerkzeug wird durch die äußere Kortikalis hindurchgestoßen und in der gegenüberliegenden Kortikalis fixiert. Dadurch ist die Achse der Bohrhülse im gleichen Abstand zum Stichwerkzeug wie die zweite Querbohrung zur ersten. Es ist dann nur noch notwendig, das Instrument um das Stichwerkzeug zu schwenken, um die Bohrerachse mit der Achse der Querbohrung im Verriegelungsnagel auszurichten.

Die Erfindung wird nachfolgend anhand von Zeichnungen näher erläutert.

Fig. 1 zeigt eine Seitenansicht, teilweise im Schnitt, eines Hilfsinstruments nach der Erfindung.

Fig. 2 zeigt einen Teil des Instruments nach Fig. 1 in Draufsicht.

Fig. 3 zeigt einen Schnitt durch das Instrument nach Fig. 1 entlang der Linie 3-3.

Fig. 4 zeigt schematisch eine andere Ausführungsform des Instruments nach der Erfindung.

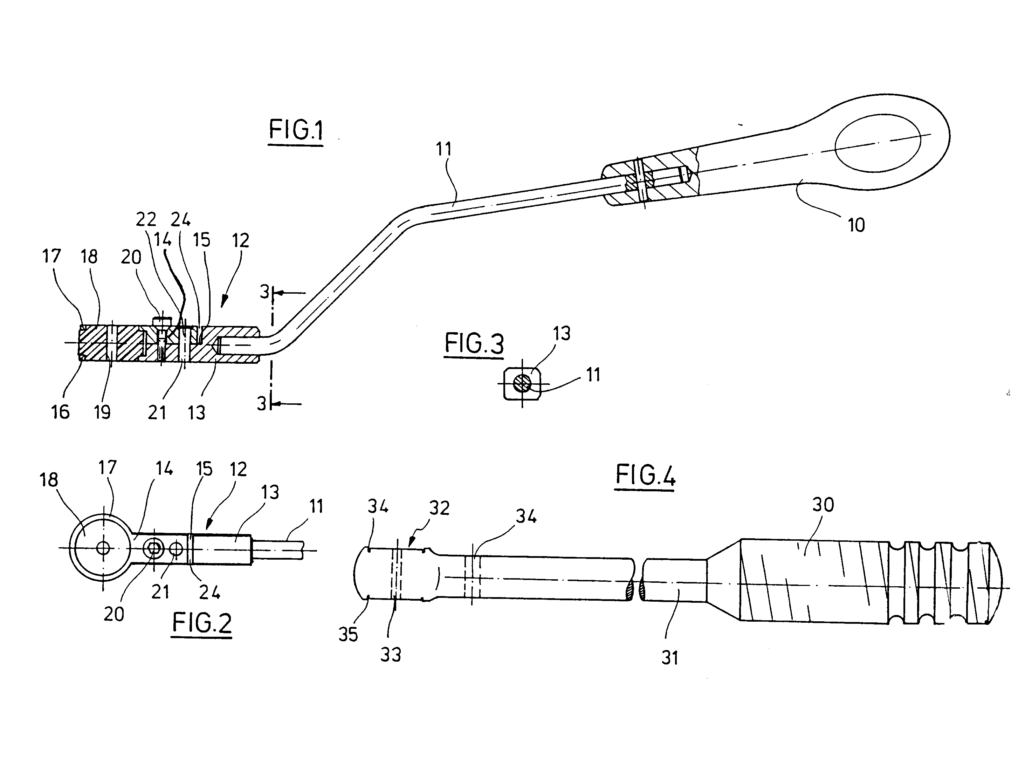

Das in Fig. 1 dargestellte Instrument besteht aus einem Handgriff 10, einem mit dem Handgriff verbundenen gekröpften Schaft 11 und einer Führungs- und Zielvorrichtung 12. Die letztere besteht aus zwei Platten 13, 14, wobei die Platte 13 mit dem Schaft 11 verbunden ist. Die Platte 13 besitzt eine abgesenkte Abflachung 15, auf die die Platte 14 aufgelegt ist, wobei die Oberseite der Platte 14 annähernd in gleicher Höhe liegt wie der rückwärtige Abschnitt der Platte 13, wie aus Fig. 1 zu erkennen. An die Platte 13 ist nach außen ein Kreisring 16 angeformt. An die Platte 14 ist ebenfalls ein Kreisring 17 angeformt. Innen- und Außendurchmesser der Ringe 16, 17 sind gleich. Man erkennt, daß die Ringe 16, 17 in kreisförmige Nuten am Rand eines kreisrunden Führungsorgans 18 einsitzen. Die Ringe 16, 17 bilden daher eine Fassung für das Führungsorgan 18. Das Führungsorgan 18 besteht aus Kunststoff, beispielsweise aus Polyamid und besitzt eine mittige Führungsbohrung 19.

Die Platten 13, 14 werden mit Hilfe einer Schraube 20 gegeneinander gespannt, wobei ein in der Platte 13 einsitzender Paßstift 21 passend in eine Bohrung 22 der Platte 14 einführbar ist.

Die Führungsbohrung 19 ist so bemessen, daß sie gleitend ein Stichwerkzeug, beispielsweise einen Steinman-Pin oder dergleichen aufnimmt. Der Stift ist jedoch mit ausreichend Reibung in der Bohrung 19 gehalten, so daß er bei der Handhabung des Instruments nicht herausfällt. Bei der Operation erfolgt zunächst eine Ausrichtung zwischen einer Querbohrung des Verriegelungsnagels und der Strahlung einer Röntgenquelle derart, daß die Querbohrung annähernd kreisförmig abgebildet ist. Dies bedeutet, daß die Achse der Querbohrung mit der Strahlenachse zusammenfällt. Anschließend wird ein in der Führungsbohrung 19 gehaltener Stift mit seiner Spitze so positioniert, daß die Spitze auf die Achse der Querbohrung des Verriegelungsnagels fällt. Hierzu wird der Stift mit seiner Spitze verhältnismäßig schräg gegen die äußere Kortikalis gehalten, um eine genaue Beobachtung der Spitze zu ermöglichen.Ist die Stelle aufgefunden,wird das Instrument um die festgelegte Spitze des Stiftes verschwenkt, und zwar so lange, bis die konzentrischen zur Führungsbohrung liegenden Ringe 16, 17 deckungsgleich auf dem Bildwandler ausgerichtet sind (die übrigen Apparaturen und auch der Verriegelungsnagel zum Setzen von Löchern für das Implantieren von Verriegelungsnägeln sind nicht gezeigt, da sie zum Stand der Technik gehören, siehe zum Beispiel EP-A-0 201 737).

Nachdem die gewünschte Ausrichtung vorgenommen worden ist, wird der Stift durch einen oder mehrere Schläge in die zugeordnete Kortikalis eingetrieben. Anschließend wird mit Hilfe eines Bohrers das gesetzte Loch aufgebohrt, wobei gleichzeitig in der gegenüberliegenden Kortikalis ebenfalls ein Loch gebohrt wird, wobei die Querbohrung im Verriegelungsnagel als Führung dient.

Die Platten 13, 14 lassen eine Nut 24 frei. Durch die Lage der Nut kann der Operateur erkennen, nach welcher Seite er das Instrument verschwenken muß, um die Ringe 16, 17 zur Deckung zu bringen.

Ist nach mehrmaligem Gebrauch die Führungsbohrung 19 zu stark aufgeweitet, werden die Platten 13, 14 voneinander getrennt. Ein neues Führungsorgan 18 kann dann zwischen die Ringe 16, 17 gesetzt werden.

Das in Fig. 4 gezeigte Instrument weist ebenfalls einen Griff 30, einen Schaft 31 sowie eine Führungs- und Zielvorrichtung 32 auf. Die Teile 30, 31 und 32 sind einteilig aus Kunststoff geformt. Eine Führungsbohrung 33 in der Vorrichtung 32 entspricht der Führungsbohrung 19 nach Fig. 1. In dem Kopf der Vorrichtung 32 sind Metallringe 34, 35 eingebettet entsprechend den Metallringen 16, 17 nach Fig. 1. Die Arbeitsweise des Instruments nach Fig. 4 entspricht der nach Fig. 1. Zusätzlich ist in einem vorgegebenen Abstand zur Führungsbohrung 33 eine weitere Bohrung 34 vorgesehen. Sie dient zur Aufnahme einer Bohrhülse. Der Abstand der Bohrungen 33, 34 entspricht dem Abstand von Querbohrungen im Verriegelungsnagel. Mit Hilfe eines Stichwerkzeugs kann das Instrument nach Fig. 4 in seiner Lage festgelegt werden, indem das Stichwerkzeug, durch die äußere Kortikalis hindurchschlagen und die Querbohrung des Verriegelungsnagels geführt, teilweise in die gegenüberliegende Kortikalis eingeschlagen wird. Anschließend braucht das Instrument nur noch um den Stift oder das Stichwerkzeug verschwenkt zu werden, bis die Achse der Bohrhülse bzw. der Bohrung 34 mit der Achse der zweiten Querbohrung im Verriegelungsnagel zusammenfällt.

## Patentansprüche

1. Hilfsinstrument zum Setzen von Löchern beim Implantieren von Verriegelungsnägeln, mit einem länglichen Schaft (11), an den am einen Ende ein Handgriff (10) und am anderen Ende eine Zielvorrichtung (12) angeordnet ist, die ein Führungsorgan (18) mit einer Führungsbohrung (19, 21) für die gleitende Führung eines spitzen Stiftes und für Röntgenstrahlung undurchlässige Zielabschnitte (16, 17) besitzt, die in Achsrichtung der Führungsbohrung (19) beabstandet sind und bei zur Führungsbohrung (19) ausgerichteter Strahlungsrichtung annähernd deckungsgleich abgebildet werden, dadurch gekennzeichnet, daß das Führungsorgan (18) aus einem für Röntgenstrahlung durchlässigen Material besteht und die Führungsbohrung (19) zur gleitenden Führung eines drahtähnlichen Stichwerkzeugs ausgebildet und zu den Zielabschnitten (16, 17) koaxial ist.

2. Instrument nach Anspruch 1, dadurch gekennzeichnet, daß die Zielabschnitte von axial beabstandeten Ringen (16, 17) gebildet sind.

3. Instrument nach Anspruch 1 oder 2, dadurch gekennzeichnet, daß das Führungsorgan (18) aus Kunststoff geformt ist und die Zielabschnitte im Kunststoff versenkt angeordnet sind.

4. Instrument nach einem der Ansprüche 1 bis 3, dadurch gekennzeichnet, daß Griff (30), Schaft (31) und Führungsorgan (32) einteilig aus Kunststoff geformt sind.

5. Instrument nach einem der Ansprüche 1 bis 4, dadurch gekennzeichnet, daß die Zielabschnitte (16, 17) als Fassung für das Führungsorgan (18) dienen.

6. Instrument nach Anspruch 5, dadurch gekennzeichnet, daß mindestens ein Zielabschnitt (16, 17) lösbar am Schaft (11) befestigbar ist.

7. Instrument nach einem der Ansprüche 1 bis 6, dadurch gekennzeichnet, daß die Zielvorrichtung (12) im Abstand zur Führungsbohrung (19) einen durchgehenden Schlitz (24) oder dergleichen aufweist, der sich quer zur Führungsbohrung (19) erstreckt.

8. Instrument nach einem der Ansprüche 1 bis 7, dadurch gekennzeichnet, daß im Abstand zur Führungsbohrung (33) eine weitere Bohrung (34) zur Aufnahme einer Bohrhülse vorgesehen ist, deren Achse parallel zur Achse der Führungsbohrung (33) verläuft.

## Claims

1. Accessory instrument for positioning implantation holes of attachment nails, comprising an elongated shank (11), having a gripping portion (10) at one end and an aiming device (12) at the other end thereof, said aiming portion including a guide member (18) having a guide bore (19,21) adapted to slidingly receive a pointed pin, and having aiming portions (16,17) impervious to X-rays, which aiming portions are spaced in the axial extension of the guide bore (19) and which aiming portions approximately appear coincident on an image when the guide bore is aligned to the beam direction,
   **characterized** in that
   the guide member (18) is made of a material transparent to X-rays, that the guide bore (19) is adapted to slidingly receive a wire-like piercing tool and that the guide bore is coaxial with respect to the aiming portions (16,17).

2. The instrument of claim 1,
   wherein said aiming portions are defined by axially spaced rings (16,17).

**3.** The instrument of claim 1 or 2,
wherein the guide member (18) is made of plastic material and the aiming portions are counter-sunk in plastic material.

**4.** The instrument of one of the claims 1 to 3,
wherein the gripping portion (30), the shank (31) and the guide member (32) are integrally molded of plastic material.

**5.** The instrument of one of claims 1 to 4,
wherein the aiming portion (16,17) serve as retaining means for the guide member (18).

**6.** The instrument of claim 5,
wherein at least an aiming portion (16,17) is releasably secured to said shank (11).

**7.** The instrument of one of claims 1 to 6,
wherein the aiming device is provided with a slot (24) or the like in spaced relation to the guiding bore (19) which slot extends transversely to the guide bore.

**8.** The instrument of one of claims 1 to 7,
wherein a further bore (34) is provided at a distance to the guide bore (32), said further bore receiving a drilling sleeve, wherein the axis of the further bore extends parallel to that of the guide bore.

**Revendications**

**1.** Instrument d'assistance au positionnement de trous pour l'implantation de clous de verrouillage, comprenant une tige allongée (11) qui porte à une extrémité une poignée (10) et à l'autre extrémité un dispositif de visée (12), qui comprend un organe de guidage (18) présentant un trou de guidage (19, 21) pour le guidage coulissant d'une pointe acérée et des parties de visée (16, 17) qui sont imperméables au rayonnement X, sont distantes dans la direction axiale du trou de guidage (19) et apparaissent approximativement comme superposées lorsque la direction de rayonnement est alignée avec le trou de guidage (19), caractérisé en ce que l'organe de guidage (18) est réalisé en un matériau perméable au rayonnement X, et le trou de guidage (19) est conçu pour le guidage coulissant d'un outil de perçage du genre fil et est co-axial aux parties de visée (16, 17).

**2.** Instrument selon la revendication 1, caractérisé en ce que les parties de visée sont constituées par des anneaux (16, 17) axialement distants.

**3.** Instrument selon la revendication 1 ou 2, caractérisé en ce que l'organe de guidage (18) est formé en matière plastique, et les parties de visée sont encastrées dans la matière plastique.

**4.** Instrument selon l'une des revendications 1 à 3, caractérisé en ce que la poignée (30), la tige (31) et l'organe de guidage (32) sont formés d'une seule pièce en matière plastique.

**5.** Instrument selon l'une des revendications 1 à 4, caractérisé en ce que les parties de visée (16, 17) servent de monture pour l'organe de guidage (18).

**6.** Instrument selon la revendication 5, caractérisé en ce qu'au moins une partie de visée (16, 17) est fixée de manière détachable sur la tige (11).

**7.** Instrument selon l'une des revendications 1 à 6, caractérisé en ce que le dispositif à visée (12) présente, à distance du trou de guidage (19), une fente traversante (24) ou similaire, qui s'étend transversalement au trou de guidage (19).

**8.** Instrument selon l'une des revendications 1 à 7, caractérisé en ce qu'un autre trou (34) est prévu à distance du trou de guidage (33) pour recevoir une douille de perçage, dont l'axe s'étend parallèlement à l'axe du trou de guidage (33).

FIG.1

FIG.3

FIG.2

FIG.4

EP 0 281 763 B1